# EUROPÄISCHE PATENTSCHRIFT

(11) **EP 0 569 383 B1**
(45) Veröffentlichungstag und Bekanntmachung des Hinweises auf die Patenterteilung: **05.06.1996**
(21) Anmeldenummer: 92902125.1
(22) Anmeldetag: 15.01.1992
(51) Int. Cl.: C08B 37/00, A61K 31/72

(54) **NIEDERMOLEKULARE SULFATIERTE POLY-$g(b)-2,6-FRUCTOFURANOSANE UND DEREN VERWENDUNG ZUR BEHANDLUNG VON DEGENERATIVEN GELENKERKRANKUNGEN**
LOW-MOLECULAR SULPHATED POLY-$g(b)-2,6-FRUCTOFURANOSANES AND THEIR USE IN THE TREATMENT OF DEGENERATIVE ARTICULAR AILMENTS
POLY-$g(b)-2,6-FRUCTOFURANOSANES SULFATES A FAIBLE POIDS MOLECULAIRE ET LEUR UTILISATION DANS LE TRAITEMENT DE MALADIES DEGENERATIVES DES ARTICULATIONS

(30) Priorität: 30.01.1991 DE 4102675
(43) Veröffentlichungstag der Anmeldung: 18.11.1993
(73) Patentinhaber: HOECHST AKTIENGESELLSCHAFT, D-65926 Frankfurt am Main (DE)
(72) Erfinder: THORWART, Werner, D-6203 Hochheim am Main (DE); RAISS, Ruth, D-6000 Frankfurt am Main 1 (DE); GRÖTSCH, Horst, D-6246 Glashütten 1 (DE); SCHLEYERBACH, Rudolf, D-6238 Hofheim am Taunus (DE)
(86) Internationale Anmeldenummer: EP9200066
(87) Internationale Veröffentlichungsnummer: WO9213895

(56) Entgegenhaltungen:
- WO-A-88/07060
- FR-M- 4 354
- GB-A- 796 737
- GB-A- 879 133

## Beschreibung

Arthrose ist eine degenerative Gelenkserkrankung mit entzündlichen Episoden und progredienter Knorpelzerstörung, die zu Funktionsbeeinträchtigung bis hin zu völliger Versteifung führen kann. Bisher sind zwar Entzündung und Schmerz bei dieser Erkrankung therapierbar, es gibt jedoch bisher kein Arzneimittel, das erwiesenermaßen den fortschreitenden Knorpelabbau aufzuhalten bzw. zu heilen vermag. Bekannte Therapeutika der Arthrose sind beispielsweise Mischungen von sulfatierten Glucoseaminoglykanen (Current Therapeutik Research, 40,6 (1986) 1034) oder nichtsteriodale Antiinflammatorika, die jedoch den Knorpelverlust nicht aufzuhalten vermögen.

Wenn auch die Pathogenese der Arthrose noch nicht im Einzelnen aufgeklärt ist, gilt als gesichert, daß die Chondrozyten (Knorpelzellen) maßgeblich an dem verstärkten Matrixverlust beteiligt sind, und daß von den Hauptbestandteilen dieser Matrix vor allem die Proteoglykane (PG) als erste enzymatisch abgebaut werden.

Erfolgsversprechende Ansatzpunkte zur Arthrosetherapie würden demnach solche Pharmaka bieten, die aufgrund ihres Wirkprofils die Proteoglykan-Synthese in Chondrozyten stimulieren und darüberhinaus einem pathologisch beschleunigten Knorpelabbau entgegenwirken.

Überraschenderweise wurde nun gefunden, daß niedermolekulare sulfatierte Poly-β-2,6-fructofuranosane die Synthese von Knorpelmatrix stimulieren, den Abbau von Knorpelmasse inhibieren und die Proteoglykan-Synthese im Knorpel wirksam erhöhen. Augrund ihrer pharmakologischen Eigenschaften eignen sie sich hervorragend zur Behandlung und Prophylaxe von degenerativen Gelenkserkrankungen, aber auch von Erkrankungen des rheumatischen Formenkreises, bei denen Knorpelabbau zu verzeichnen ist, wie bei der chronischen Polyarthritis, dem Gelenktrauma sowie bei Knorpelschwund nach längerer Immobilisation des Gelenks.

In der deutschen Patentschrift 1 300 668 sind einige der erfindungsgemäß verwendbaren Verbindungen beschrieben worden, doch über ihre Verwendung bei degenerativen Gelenkserkrankungen ist nichts bekannt.

Die Erfindung betrifft daher sulfatierte Poly-β-2,6-fructofuranosane, die Monomereinheiten der Formel I enthalten, 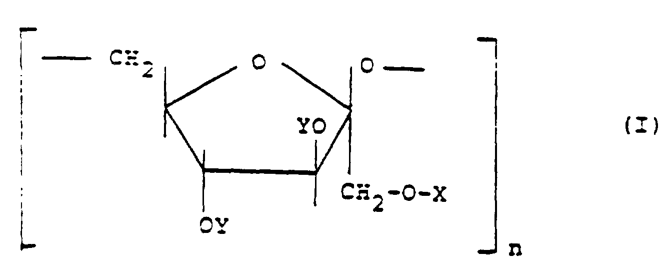 wobei
- X: für
a) -SO₃M oder
b) -(CH₂)ₘ-SO₃M,
M für ein physiologisch verträgliches Kation oder Wasserstoff,
m für eine ganze Zahl von 2 bis 4,
- Y: für
a) Wasserstoff,
b) -SO₃M oder
c) -(CH₂)ₘ-SO₃M,
wobei M und m die obengenannte Bedeutung haben und
- n: für eine ganze Zahl von 10 bis 200
steht und der Sulfatierungsgrad zwischen 1 und 2 liegt.

Bevorzugt sind die Verbindungen der Formel I, in denen n für eine ganze Zahl von 15 bis 75 steht. Ganz besonders bevorzugt sind die Verbindungen der Formel I in denen n für eine ganze Zahl von 20 bis 30 steht.

Geeignete physiologisch verträgliche Kationen sind beispielsweise Alkali-, Erdalkali- und Ammoniumkationen, einschließlich solcher von organischen Ammoniumbasen.

Unter dem Begriff Sulfatierungsgrad wird das Molverhältnis zwischen Sulfogruppe und Zuckermonomereinheit der Formel I (Fructose) verstanden.

Die erfindungsgemäßen Verbindungen der Formel I können ein mittleres Molekulargewicht aufweisen, das einen weiten Bereich überstreicht. Vorzugsweise haben die erfindungsgemäßen Verbindungen ein mittleres Molekulargewicht von 3000 bis 65000, besonders bevorzugt von 3000 bis 20000, insbesondere von 5000 bis 12000.

Die Herstellung der erfindungsgemäßen Verbindungen erfolgt beispielsweise durch Behandlung von Poly-β-2,6-fructofuranosanen (Laevanen) mit Chlorsulfonsäure, vorzugsweise in Gegenwart einer Base, wie Pyridin. Zweckmäßig arbeitet man in Gegenwart eines Lösungsmittels wie Formamid. Bei dieser Arbeitsweise fällt der Laevan-Schwefelsäureester in Form eines Salzes an, das dann gegebenenfalls nach Umwandlung in ein Metallsalz, z.B. das Natriumsalz, der nachfolgenden Hydrolyse unterworfen wird.

Die Hydrolyse kann in bekannter Weise durch Einstellen des pH-Wertes einer Lösung eines Salzes eines hochmolekularen Laevan-Schwefelsäureesters auf einen pH-Wert unterhalb etwa 2,5 vorgenommen werden. Am einfachsten geschieht das durch Versetzen der Lösung mit einer geeigneten, d.h. hinreichend starken Säure, z.B. einer Mineralsäure wie Salzsäure oder Schwefelsäure in einer solchen Menge, daß der genannte pH-Wert erreicht wird.

Auf besonders einfache und schonende Weise kann die Hydrolyse der Salze hochmolekularer Laevan-Schwefelsäureester ausgeführt werden, indem man deren wäßrigen Lösungen einen Sulfogruppen enthaltenden Kationenaustauscher zusetzt. Dieser zeigt die gleiche Wirkung wie eine freie starke Säure, besitzt jedoch dieser gegenüber den Vorteil, daß keine Fremdionen in die Lösung gelangen. Geeignete lonenaustauscher sind beispielsweise unter den geschützten Bezeichnungen Amberlite IRC 120, IRC 112, IRC 105, Dowex 50 oder Leuatit KS im Handel erhältlich.

Da die lonenaustauscher die Lösungen in verhältnismäßig kurzer Zeit sauer stellen, noch bevor die Hydrolyse der Laevan-Schwefelsäureester ein merkliches Ausmaß angenommen hat, kann man sie schon nach kurzer Zeit, etwa nach 2 bis 5 Minuten aus der Lösung entfernen, ohne daß dadurch der Ablauf der Hydrolyse beeinflußt wird. Andererseits kann der lonenaustauscher aber auch bis zum Abschluß der Hydrolyse in der Lösung verbleiben, ohne daß dadurch eine Störung in deren Ablauf eintritt.

Die Hydrolyse wird zweckmäßig bei erhöhter Temperatur zwischen 30 und 70°C, vorzugsweise bei etwa 50 bis 60°C, und etwa 10 bis 100 Minuten lang ausgeführt.

Der Umfang des hydrolytischen Abbaues kann durch Herabsetzung des pH-Wertes, Verlängerung der Hydrolysedauer oder Erhöhung der Temperatur der Lösung vergrößert oder durch die umgekehrten Maßnahmen verkleinert werden.

Durch Wahl der geeigneten Bedingungen in dem hier gesteckten Rahmen, die nötigenfalls durch Vorversuche ermittelt werden können, lassen sich Laevan-Schwefelsäureester mit vorherbestimmten Molekulargewichten herstellen.

Durch Veränderung des Chlorsulfonsäure/Laevan-Molverhältnisses lassen sich Laevane herstellen bei denen der Sulfatierungsgrad pro Zuckereinheit kleiner als 2 sein kann. So können beispielsweise durch die Wahl des Chlorsulfonsäure/Laevan-Molverhältnisses von 2,5 zu 1, Laevane mit einem Sulfatierungsgrad pro Zuckereinheit von 1,6 erhalten werden.

Die hochmolekularen Laevane können auch zuerst nach bekannten Verfahren, beispielsweise durch Hydrolyse mit Salzsäure, in niedermolekulare Laevane überführt werden, die anschließend mit bekannten (DE 1 300 668) oder mit den obengenannten Methoden sulfatiert werden.

Zur Darstellung der erfindungsgemäßen Verbindungen, in denen für den Rest X und gegebenenfalls einen der beiden Reste Y eine Sulfatoalkyl-Gruppe steht, werden kürzerkettige Laevan-Fragmente mit einer Halogenalkylsulfonsäure, wie beispielsweise 1-Chloräthansulfonsäure oder einem Sulton wie 1,3-Propan- oder 1,4-Butansulton in Gegenwart einer starken Base z.B. Natrium- oder Kaliumhydroxid umgesetzt.

Eine bevorzugte Ausführungsform dieser Verfahrensweise besteht in der Umsetzung eines Sultons mit Laevan-Fragmenten im Molverhältnis 2:1 in einer Lösung von Natriumhydroxid in einem Alkohol wie z.B. Methanol oder Ethanol bei der Siedetemperatur des Lösungsmittels.

Die vorliegende Erfindung betrifft weiterhin die Verwendung von sulfatierten Poly-β-2,6-fructofuranosanen, wobei ein Sulfatierungsgrad von 2 nicht ausgenommen ist, zur Prophylaxe und Behandlung degenerativer Gelenkserkrankungen. Dazu gehören z.B. Arthrosen, sonstige Erkrankungen des rheumatischen Formenkreises mit Knorpelabbau, chronischer Polyarthritis, Knorpelschwund nach Gelenktrauma, beispielsweise nach Meniskus- oder Pattellaverletzungen oder Bänderrissen, oder bei Knorpelschwund nach längerer Stillegung von Gelenken. Die erfindungsgemäßen Polysaccharide können auf unterschiedliche Weise verabreicht werden. Sie können beispielsweise oral, intramuskulär, periartikulär, intraartikulär, intravenös, intraperitoneal, subkutan oder rektal verabreicht werden.

Die Erfindung betrifft auch Arzneimittel, gekennzeichnet, durch einen wirksamen Gehalt von mindestens einem erfindungsgemäß sulfatierten Poly-β-2,6-fructofuranosan neben üblichen pharmakologisch verträglichen Träger- und/oder Zusatzstoffen.

Die Herstellung der erfindungsgemäßen Arzneimittel erfolgt, indem mindestens ein sulfatiertes Poly-β-2,6-fructofuranosan gegebenenfalls mit weiteren Hilfs- und/oder Trägerstoffen in eine geeignete Darreichungsform gebracht wird. Die Hilfs- und Trägerstoffe stammen aus der Gruppe der Trägermittel, Konservierungsstoffe und anderer üblicher Hilfsstoffe.

Zum Beispiel können für orale Darreichungsformen Hilfsstoffe wie Stärken, z.B. Kartoffel-, Mais- oder Weizenstärke, Cellulose bzw. deren Derivate, insbesondere mikrokristalline Cellulose, Siliziumdioxid, verschiedene Zucker wie Milchzucker, Magnesiumcarbonat und/oder Calciumphosphate verwendet werden. Weiterhin ist es vorteilhaft, den oralen Darreichungsformen Hilfsstoffe zuzusetzen, die die Verträglichleit der Medikamente verbessern, wie z.B. Schleimbildner und Harze. Zwecks besserer Verträglichkeit können die Medikamente auch in Form von magensaftunlöslichen Kapseln verabreicht werden. Darüber hinaus kann es vorteilhaft sein, der Darreichungsform, bzw. einer Komponente des Kombinationspräparates, ein Retardierungsmittel zuzusetzen, gegebenenfalls in Form von permeablen Membranen, wie z.B. solche auf Cellulose- oder Polystyrolharzbasis oder lonenaustauscher.

Die anzuwendende Dosierung der erfindungsgemäßen Arzneimittel ist abhängig von verschiedenen Faktoren wie Darreichungsform des Medikamentes und Zustand, Gewicht und Art der Erkrankung des Patienten. Eine Tagesdosis von etwa 100 bis 3000 mg des erfindungsgemäßen Poly-β-2,6-fructofuranosans, vorzugsweise 300 bis 1500 mg sind indiziert. Unter Umständen können jedoch auch höhere oder niedrigere Tagesdosen angebracht sein. Die Verabreichung der Tagesdosis der erfindungsgemäßen Polysaccharide kann in Form einer einzelnen Verabreichung oder in mehreren kleinen Dosen erfolgen. Bevorzugt ist die Verabreichung in 2 bis 4 Dosen pro Tag.

### Beispiele:

### Vorschrift 1a:

Disulfatierte Poly-β-2,6-fructofuranosane der allgemeinen Formel I, bei denen der x-und mindestens einer der beiden y-Reste eine NaO₃S-Gruppe darstellt:

Zu einer Lösung von 670 ml Pyridin und 400 ml Chloroform werden unter kräftigem Rühren innerhalb von 2 Stunden 227 ml (3,3 mol) Chlorsulfonsäure zugetropft, wobei die Reaktionstemperatur unter 10°C gehalten wird. Nach Bildung des Pyridin/Schwefeltrioxid-Komplexes werden bei 10°C 800 ml Formamid zugegeben und weitere 15 Minuten gerührt. Nach dem Eintragen von 162 g Laevan, erhitzt man die viskose Reaktionsmischung 15 Minuten auf Rückflußtemperatur. Nach erfolgter Sulfatierung wird die Mischung auf 40-45°C abgekühlt und unter Rühren zu 6000 ml Methanol gegeben. Dabei fällt das Pyridiniumsalz der Laevansulfonsäure als schmieriger Niederschlag aus, der nach Entfernen der überstehenden Lösungsmittel noch zweimal mit jeweils 300 ml Methanol nachgewaschen wird.

Zur Überführung in das Dinatriumsalz wird die zähe Masse in 1200 ml Wasser gelöst und mit 50 %iger Natronlauge auf pH 10 eingestellt. Beim anschließenden Eingießen der Lösung in 3500 ml Methanol scheidet sich das Natriumsalz ab, das nochmals nach Abfiltrieren mit 2000 ml Methanol nachgewaschen wird. Zur Verbesserung der Reinheit wird diese Umfällung wiederholt und abschließend das Produkt im Vakuum bei 50°C getrocknet.
Ausbeute: 320 g (87 % d.Th.)
MG: (C₆H₈Na₂O₁₁S₂)ₙ = (366,2)ₙ
Analyse: S_{ber} = 17,51; S_{gef} = 17,47

### Vorschrift 1b:

Abbau der hochpolymeren disulfatierten Laevane zu Verbindungen der Formel I, in denen n eine Zahl zwischen 15 und 60 darstellt:

Eine Lösung von 100 g der hochpolymeren disulfatierten Laevane aus Beispiel 1a) in 500 ml Wasser wird mit ca. 6,4 g des Kationenaustauschers Amberlite IR C120 auf pH 2,5 eingestellt. Nach Stabilisierung des pH-Wertes wird das Austauscherharz abgesaugt und das Filtrat im Wasserbad unter Rühren auf 60°C erwärmt. Dabei bestimmt die Zeit der Erwärmung den Grad des Abbaus (vgl. Tabelle 1), wobei das Fortschreiten des Laevanabbaus mittels Viskosimeter kontrolliert wird. Nach Erreichen der gewünschten Viskosität, kühlt man die Lösung ab, stellt sie mit 50 %iger Natronlauge auf pH 6,5-6,8 ein und engt sie im Vakuum auf ein Drittel ihres Volumens ein. Danach gießt man die Lösung in 3000 ml Methanol, der noch 30 ml einer gesättigten Natriumchlorid-Lösung zugesetzt wird. Der sich bildende Niederschlag wird auf gleiche Weise nochmals umgefällt, abgesaugt und im Vakuum bei 50°C getrocknet. Die Ergebnisse werden in Tabelle 1 gezeigt.

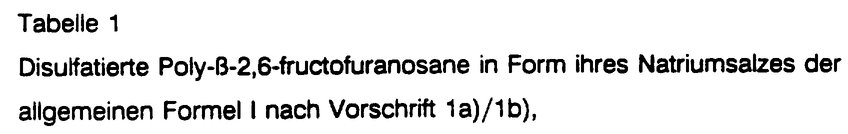

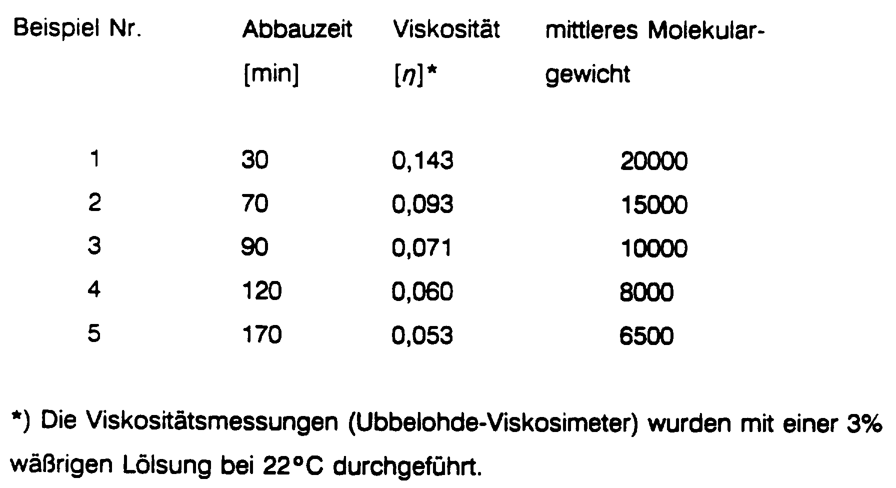

### Vorschrift 2a:

Sulfatierte Poly-β-2,6-fructofuranosane der allgemeinen Formel I, bei denen der Rest x und einer der beiden y-Reste partiell eine NaO₃S-Gruppe darstellt:

In Analogie zur Vorschrift 1a) erhält man durch die Wahl des Chlorsulfonsäure/Laevan-Molverhältnis von 2,5:1, solche Leavane, bei denen der Gesamt-Sulfatierungsgrad pro Zuckereinheit bei 1,6 liegt, wozu bezogen auf die Vorschrift 1a), 166 ml Chlorsulfonsäure benötigt werden.
Ausbeute: 248 g (76 % d. Th.)
Analyse: S_{ber}: 14,99 %, S_{gef}: 14,77 %

### Vorschrift 2b:

Der Abbau des nach Vorschrift 2a) dargestellten hochpolymeren sulfatierten Laevans erfolgt auf gleiche Weise wie in Vorschrift 1b) beschrieben. Die dabei entstandenen niedermolekularen Fraktionen, in denen n eine Zahl zwischen 30 und 60 darstellt, sind in Tabelle 2 aufgeführt.

**Tabelle 2**

| Beispiel Nr. | Abbauzeit [min] | Viskosität [η]* | mittleres Molekulargewicht |
|---|---|---|---|
| 6 | 11 | 0,135 | 20000 |
| 7 | 13 | 0,078 | 12000 |
| 8 | 20 | 0,069 | 10000 |

### Vorschrift 3a:

Monosulfatierte Poly-β-2,6-fructofuranosane der allgemeinen Formel I, bei denen der Rest x eine NaO₃S-Gruppe darstellt:

In Analogie zur Vorschrift 1a) erhält man monosulfatiertes Laevan, wenn Chlorsulfonsäure zu Laevan im Molverhältnis 2:1 eingesetzt wird. Dazu werden bezogen auf Vorschrift 1a) 133 ml (2 Mol) Chlorsulfonsäure benötigt.
Ausbeute: 214 g (81 % d. Th.)
MG: (C₆H₉NaO₈S)ₙ = (264,2)ₙ
Analyse: S_{ber}: 12,14 %, S_{gef}: 12,6 %

### Vorschrift 3b:

Der Abbau des hochpolymeren monosulfatierten Laevans zu Verbindungen der Formel I erfolgt auf gleiche Weise wie in Vorschrift 1b). Die dabei entstandenen niedermolekularen Fraktionen sind in Tabelle 3 aufgeführt.

**Tabelle 3**

| Beispiel Nr. | Abbauzeit [min] | Viskosität [η]* | mittleres Molekulargewicht |
|---|---|---|---|
| 9 | 8 | 0,318 | 20000 |
| 10 | 20 | 0,089 | 10000 |
| 11 | 30 | 0,055 | 8000 |

### Vorschrift 4:

Darstellung von Di-(3-sulfonatopropyl)-poly-β-2,6-fructo-furanosan-Natriumsalz, in denen die Sulfonatopropyl-Gruppe für den x- und einen der beiden y-Reste steht:

### 4a) Hydrolyse des hochpolymeren Laevans

Zu einer Lösung von 3,6 ml konzentrierter Salzsäure in 600 ml Wasser werden 120 g Laevan gegeben und bei 25°C 4,5 Stunden gerührt. Nach Neutralisation mit 50 %iger Natronlauge wird die Lösung in 7000 ml Methanol gegossen, wobei der sich dabei bildende wachsartige Niederschlag abgesaugt und bei 60°C im Vakuumtrockenschrank getrocknet wird.
Ausbeute: 80 g
Viskosität: [η] = 0,122 (3 % Lösung in Wasser bei 22°C)

4b) Di-(3-sulfonatopropyl)-poly-β-2,6-fructo-furanosan-Natriumsalz (Beispiel 12) Zu einer Lösung von 10 g (0,25 mol) Natriumhydroxid in 800 ml Methanol werden nacheinander zuerst 20,3 g (0,125 mol) feingepulvertes Laevan aus Vorschrift 4a) und dann 30,5 g (0,25 mol) 1,3-Propansulton zugegeben. Anschließendes 5 stündiges Erhitzen der Mischung unter Rückfluß führt zu vollständiger Lösung. Beim langsamen Abkühlen bildet sich ein Niederschlag, der abfiltriert und mit Methanol nachgewaschen wird. Das verbleibende Filtrat wird auf ca. 150 ml Flüssigkeit eingeengt und unter kräftigem Rühren in 1200 ml Essigsäureethylester gegossen. Die sich abscheidenden Kristalle werden abfiltriert und im Vakuum bei 60°C getrocknet.
Ausbeute: 34,3 g (61 % d. Th.)
Schmelzpunkt: 164-165°C (Zersetzung)
MG: (C₁₂H₂₀Na₂O₁₁S₂)ₙ = (450,4)ₙ, wobei die Zahl n zwischen 20 und 30 liegt.
Analyse: S_{ber} = 14,2 %, S_{gel} = 14,7 %
Viskosität: 0,033 (3 % wäßrige Lösung bei 22°C)

Die Wirksamkeit der erfindungsgemäßen Polysaccharide wird in folgenden Versuchen nachgewiesen.

### 1. Wirksamkeit in der Synthese-Stimulierung von Knorpel-Matrix, Prüfung in der Chondrozyten-Kultur

Zellen: Den Fußgelenken frisch geschlachteter Rinder werden der hyaline Knorpel entnommen, mit Pronase (Boehringer Mannheim) und Collagenase (Sigma), die native Matrix enzymatisch abgebaut, und die Chondrozyten in 1 %iger "low melting Agarose" in 24er Multiwell-Schalen in einer Zelldichte von 4 x 10⁶ pro well ausplatiert.

Medium: Komplettes Medium enthält HAM's F12 (Biochrom KG, Berlin) und 10 % foetales Kälberserum (Boehringer Mannheim), die Testsubstanz wird in Medium gelöst, üblicherweise in einer Konzentration von 10⁻⁵ M zugegeben und bei jedem Mediumwechsel erneut zugefügt.

Versuchs-Durchführung: Die Behandlung erfolgt vom dritten bis zehnten Tag der Primärkultur, am 9. Tag wird 20 µC/ml (7,4 x 10⁵ Bq) Na₂³⁵SO₄ für 24 h dem Medium zugefügt.

Die dissoziative Extraktion der Proteoglykane aus der Agarose-Schicht wird mit 8M Guanidinium-Hydrochlorid und beigefügten Proteinase-Inhibitoren (Sigma) unter Schütteln bei 4°C über 24 h durchgeführt. Der Überstand wird nach Zentrifugation über eine PD 10 ®Sephadex G 25 Säule in freies und inkorporiertes Sulfat getrennt, dessen Aktivität nach Aliquotierung im β-Szintillationscounter gemessen wird.

Auswertung: Der Parameter für die Matrixproduktion der Chondrozyten ist die Menge synthetisierter Proteoglykane, gemessen als Sulfatinkorporation in cpm. Aus vier wells pro Gruppe wird der Mittelwert errechnet und als Prozentwert bezogen auf die unbehandelte Kontrollgruppe = 100 % angegeben.
Ergebnisse: Die Ergebnisse sind in Tabelle 4 aufgeführt.

**Tabelle 4:**

| Stimulierung der Proteoglykan-Synthese in der Agarose-Zellkultur | |
|---|---|
| Präparat | % ³⁵SO₄-Inkorporation bezogen auf unbehandelte Kontrolle = 100 % |
| Verbindung nach Beispiel Arteparon ** | 96-140* |
| 4 | 183-282* |
| 5 | 139-184* |
| 6 | 181-255* |
| 7 | 125-205* |
| 8 | 177-204* |
| 9 | 130-155* |
| 10 | 108-152* |
| 11 | 108-190* |
| 12 | 100 |

| | |
|---|---|
| * Bereich der Mittelwerte mehrerer gleichartiger Versuche | |
| ** Luitpold-Werk, München, FRG | |

### 2. Wirksamkeit in der chondrozytären Chondrolyse, Prüfung in der Chondrozyten-Kultur

Zellen werden gewonnen und platiert wie in Experiment 1

Medium: Dem kompletten Medium wie in Test 1 wird zusätzlich ab Behandlungsbeginn 5 u/ml humanes rec. Interleukin-1 alpha (IL-1, Sigma) zugesetzt, das wie die Testsubstanz bei jedem Mediumwechsel erneut zugefügt wird.

Versuchs-Durchführung: Die Behandlung erfolgt vom 5. bis 13. Tag der Primärkultur, am 12. Tag wird 20 µC/ml Na₂³⁵SO₄ für 24 h dem Medium zugefügt.

Die dissoziative Extraktion der Proteoglykane sowie Messung der Sulfatinkorporation wird wie in Test 1 durchgeführt.

Auswertung: IL-1 führt zu einer Synthesehemmung sowie zu einer Degradationssteigerung der Proteoglykane, was sich im geringeren Gehalt an markierten Matrixmolekülen in der Agarose-Schicht widerspiegelt. Die Prozentwerte werden berechnet wie in Test 1.

Ergebnisse: Die Ergebnisse sind in Tabelle 5 aufgeführt.

**Tabelle 5**

| Einfluß auf IL-1-induzierte Chondrolyse in der Agarose-Zellkultur | |
|---|---|
| Präparat | % ³⁵SO₄-Inkorporation bezogen auf unbehandelte Kontrolle = 100 % |
| IL-1 + Verbindung nach Beispiel | |
| IL-1 Kontrolle | 65 |
| IL-1 + Arteparon | 103 |
| IL-1 + 4 | 150 |
| IL-1 + 5 | 169 |
| IL-1 + 6 | 99 |
| IL-1 + 7 | 189 |
| IL-1 + 8 | 158 |
| IL-1 + 9 | 124 |
| IL-1 + 10 | 126 |
| IL-1 + 11 | 126 |
| IL-1 + 12 | 88 |

### 3. Wirksamkeit in der Synthese-Stimulierung von Knorpel. Prüfung in der Knorpel-Explant-Kultur

Knorpelgewebe: Aus den Fußgelenken frisch geschlachteter Rinder werden genormte Scheiben hyalinen Knorpels ausgestanzt und in 24er Multiwell-Schalen mit ca. 200 mg pro well kultiviert.

Medium: Komplettes Medium enthält HAM's F12 und 10 % foetales Kälberserum, die Testsubstanz wird in Medium gelöst, üblicherweise in einer Konzentration von 10⁻⁵ M zugegeben und bei jedem Mediumwechsel erneut zugefügt.

Versuchs-Durchführung: Die Behandlung erfolgt vom dritten bis zehnten Tag der Explantkultur, am 9. Tag wird 20 µC/ml Na₂³⁵SO₄ für 24 h dem Medium zugefügt.

Die dissoziative und anschließend assoziative Extraktion der Proteoglykane aus den Knorpelexplanten wird mit 8M Guanidinium-Hydrochlorid und beigefügten Proteinase-Inhibitoren unter mehrfachem Tieffrieren und Auftauen durchgeführt. Der Überstand wird nach Zentrifugation über eine PD 10 Sephadex G 25 Säule in freies und inkorporiertes Sulfat getrennt, dessen Aktivität nach Aliquotierung im β-Szintillationscounter gemessen wird.

Auswertung: Der Parameter für die Matrixproduktion der Chondrozyten ist die Menge synthetisierter Proteoglykane, gemessen als Sulfatinkorporation in cpm. Aus sechs wells pro Gruppe wird der Mittelwert errechnet und als Prozentwert bezogen auf die unbehandelte Kontrollgruppe = 100 % angegeben.

Ergebnisse: Die Ergebnisse sind in Tabelle 6 aufgeführt.

**Tabelle 6**

| Stimulierung der Proteoglykan-Synthese in der Knorpel-Explantkultur | |
|---|---|
| Präparat | % ³⁵SO₄-Inkorporation bezogen auf unbehandelte Kontrolle = 100 % |
| Verbindung nach Beispiel Arteparon | 121 |
| 9 | 147 |
| 10 | 188 |
| 11 | 205 |

## Patentansprüche

1. Sulfatierte Poly-β-2,6-fructofuranosane, die Monomereinheiten der Formel I enthalten 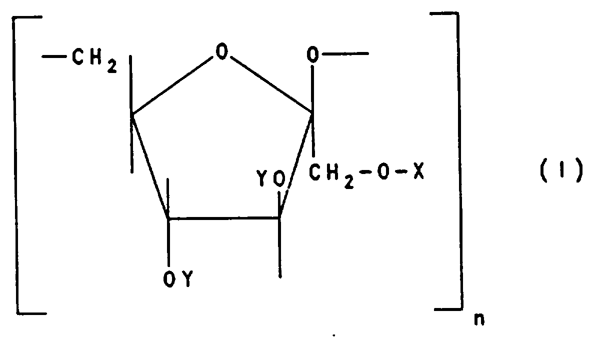 wobei
X für
a) -SO₃M oder
b) -(CH₂)ₘ-SO₃M,
M für ein physiologisch verträgliches Kation oder Wasserstoff,
m für eine ganze Zahl von 2 bis 4,
Y für
a) Wasserstoff
b) -SO₃M oder
c) -(CH₂)ₘ-SO₃M,
wobei M und m die obengenannte Bedeutung haben und
n für eine ganze Zahl von 15 bis 75 steht und das Molverhältnis zwischen Sulfogruppe und Zuckermonomereinheit der Formel I zwischen 1 und 2 liegt.

2. Sulfatierte Poly-β-2,6-fructofuranosane gemäß Anspruch 1, dadurch gekennzeichnet, daß n für eine ganze Zahl von 20 bis 30 steht.

3. Arzneimittel gekennzeichnet durch einen wirksamen Gehalt von mindestens einem sulfatierten Poly-β-2,6-fructofuranosans gemäß Anspruch 1 oder 2 neben üblichen pharmakologisch verträglichen Träger- und/oder Zusatzstoffen.

4. Arzneimittel, zur Prophylaxe und Behandlung von degenerativen Gelenkserkrankungen, gekennzeichnet durch einen wirksamen Gehalt an einer oder mehrerer der Verbindungen gemäß Anspruch 1 oder 2, wobei Polysaccharide mit einem Molverhältnis zwischen Sulfogruppe und Zuckermonomereinheit der Formel I von 2 eingeschlossen sind, neben üblichen pharmakologisch verträglichen Träger- und/oder Zusatzstoffen.

5. Verwendung von Verbindungen gemäß Anspruch 1 oder 2, wobei Polysaccharide mit einem Molverhältnis zwischen Sulfogruppe und Zuckermonomereinheit der Formel I von 2 eingeschlossen sind, zur Herstellung von Arzneimitteln zur Prophylaxe und Behandlung von degenerativen Gelenkserkrankungen.

6. Verfahren zur Herstellung von Arzneimitteln zur Prophylaxe und Behandlung von degenerativen Gelenkserkrankungen, dadurch gekennzeichnet, daß ein oder mehrere substituierte Polysaccharide gemäß Anspruch 1 oder 2, wobei Polysaccharide mit einem Molverhältnis zwischen Sulfogruppe und Zuckermonomereinheit der Formel I von 2 eingeschlossen sind, mit geeigneten Hilfs- und/oder Trägerstoffen in eine geeignete Darreichungsform gebracht werden.

## Claims

1. A sulfated poly-β-2,6-fructofuranosan which contains monomer units of the formula I 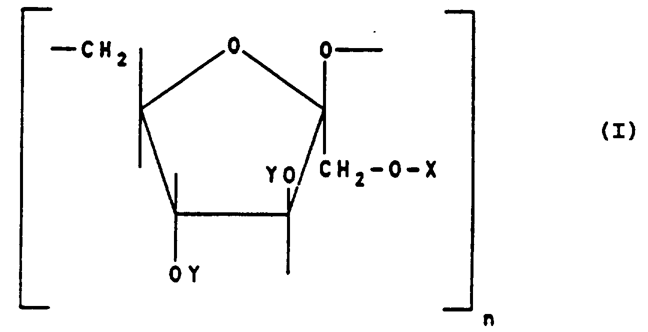 where
X is
a) -SO₃M or
b) -(CH₂)ₘ-SO₃M,
M is a physiologically tolerated cation or hydrogen,
m is an integer from 2 to 4,
Y is
a) hydrogen
b) -SO₃M or
c) -(CH₂)ₘ-SO₃M,
where M and m have the abovementioned meaning, and
n is an integer from 15 to 75 and the molar ratio between sulfo group and sugar monomer unit of the formula I is between 1 and 2.

2. A sulfated poly-β-2,6-fructofuranosan as claimed in claim 1, wherein n is an integer from 20 to 30.

3. A pharmaceutical which has an effective content of at least one sulfated poly-β-2,6-fructofuranosan as claimed in claim 1 or 2 besides customary pharmacologically acceptable excipients and/or additives.

4. A pharmaceutical for the prophylaxis and treatment of degenerative joint disorders, which has an effective content of one or more of the compounds as claimed in claim 1 or 2, where polysaccharides with a molar ratio between sulfo group and sugar monomer unit of the formula I of 2 are included, besides customary pharmacologically acceptable excipients and/or additives.

5. The use of compounds as claimed in claim 1 or 2, where polysaccharides with a molar ratio between sulfo group and sugar monomer unit of the formula I of 2 are included, for the production of pharmaceuticals for the prophylaxis and treatment of degenerative joint disorders.

6. A process for the production of pharmaceuticals for the prophylaxis and treatment of degenerative joint disorders, which comprises converting one or more substituted polysaccharides as claimed in claim 1 or 2, where polysaccharides with a molar ratio between sulfo group and sugar monomer unit of the formula I of 2 are included, with suitable auxiliaries and/or excipients into a suitable dosage form.

## Revendications

1. Poly-β-2,6-fructofuranosanes sulfates, contenant des motifs de formule I 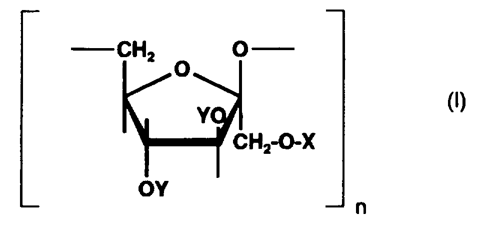 où
X représente
a) -SO₃M ou
b) -(CH₂)ₘ-SO₃M
où M est un cation physiologiquement acceptable ou un atome d'hydrogène
et m un entier de 2 à 4,
Y représente
a) un atome d'hydrogène,
b) -SO₃M ou
c) -(CH₂)ₘ-SO₃M,
où M et n ont la même signification que précédemment et n est un entier de 15 à 75 et le rapport molaire entre les groupes sulfo et les motifs monomères de glucide est compris entre 1 et 2.

2. Poly-β-2,6-fructofuranosanes sulfates selon la revendication 1, caractérisés en ce que n est un nombre entier de 20 à 30.

3. Médicament caractérisé par une quantité efficace d'au moins un poly-β-2,6-fructofuranosane sulfate selon la revendication 1 ou 2, en plus des véhicules et/ou des composés additionnels courants pharmacologiquement acceptables.

4. Médicament pour la prophylaxie et le traitement de maladies dégénératives des articulations, caractérisé par une quantité efficace d'au moins un poly-β-2,6-fructofuranosane sulfate selon la revendication 1 ou 2, incluant les polysaccharides dont le rapport molaire entre les groupes sulfo et les motifs monomères de glucide de formule I de 2, en plus des véhicules et/ou des composés additionnels courants pharmacologiquement acceptables.

5. Utilisation des composés correspondants à la revendication 1 ou 2, incluant les polysaccharides dont le rapport molaire entre les groupes sulfo et les motifs monomériques de glucide de formule I est de 2, pour la fabrication de médicaments pour la prophylaxie et le traitement de maladies dégénératives des articulations.

6. Procédé de fabrication de médicaments pour la prophylaxie et le traitement de maladies dégénératives des articulations, caractérisé en ce que l'on met sous une forme appropriée de présentation, un ou plusieurs des polysaccharides substituées correspondants à la revendication 1 ou 2, incluant les polysaccharides dont le rapport molaire entre les groupes sulfo et les motifs monomères de glucide de formule I est de 2, avec des véhicules et/ou des composés additionnels appropriés.
